# EUROPEAN PATENT SPECIFICATION

(11) **EP 1 776 343 B1**
(45) Date of publication and mention of the grant of the patent: **04.03.2009**
(21) Application number: 05768021.7
(22) Date of filing: 28.07.2005
(51) Int. Cl.: C07D 215/52, A61K 31/47, A61P 25/18

(54) **QUINOLINE DERIVATIVES AS NEUROKININ RECEPTOR ANTAGONISTS**
CHINOLINDERIVATE ALS NEUROKININREZEPTOR-ANTAGONISTEN
DÉRIVÉS DE QUINOLEINE TELS QUE LES ANTAGONISTES DU RÉCEPTEUR DE LA NEUROKININE

(30) Priority: 06.08.2004 GB 0417559
(43) Date of publication of application: 25.04.2007
(73) Proprietor: MERCK SHARP & DOHME LTD., Hoddesdon, Hertfordshire EN11 9BU (GB)
(72) Inventor: CARLING, William Robert, Harlow Essex CM20 2QR (GB)
(74) Representative: Man, Jocelyn
(86) International application number: PCT/GB2005/050121
(87) International publication number: WO 2006/013394

(56) References cited:
- WO-A-20/04050627
- US-A1- 2003 236 281
- WERMUTH ET AL: PRACTICE OF MEDICINAL CHEMISTRY, 1996, pages 203-237, XP002190259
- SAUDI, MANAL N. S. ET AL: "Synthesis of 2-(4-biphenylyl)quinoline-4-carboxylate and carboxamide analogs. New human neurokinin -3 (hNK-3) receptor antagonists" ARCHIV DER PHARMAZIE, vol. 336, no. 3, 2003, pages 165-174, XP009055980

## Description

The present invention relates to substituted quinoline-4-carboxylic acid hydrazides defined herein, pharmaceutical compositions comprising them and their use in treating diseases mediated by neurokinin-2 and/or neurokinin-3 (NK-3) receptors. These compounds can thus be used in methods of treatment to suppress and treat such disorders.

Background information on NK-3 receptor antagonists can be found in literature reviews such as Giardina and Raveglia, Exp. Opin. Ther. Patents (1997) 7(4): 307-323 and Giardina et al., Exp. Opin. Ther. Patents (2000) 10(6): 939-960. These references also contain pertinent information on preclinical validation of therapies that can be treated with NK-3 antagonists.

Representative examples of compounds prepared in the art as NK-3 antagonists are to be found in WO-A-9719926 (SmithKline Beecham S.p.a.) and US-A-5741910 (Sanofi). Structurally related compounds as NK-3 and/or NK-2 receptor antagonists are disclosed in published International patent application nos. WO2004/050626 and WO2004/050627 (both SmithKline Beecham Corporation) and International patent application no. PCT/GB2004/000415 (Merck Sharp & Dohme).

The present invention thus provides a compound of Formula (I): 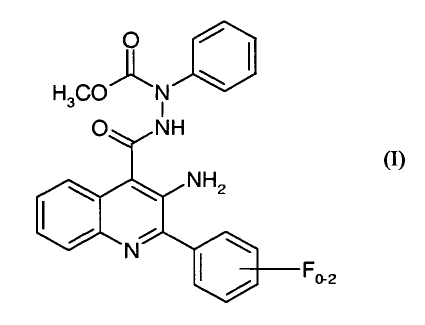 or a pharmaceutically acceptable salt thereof.

Preferably, the 2-position of the phenyl ring (where said phenyl ring is attached to the 2-position of the quinolinyl moiety) is unsubstituted. More preferably, said phenyl ring is unsubstituted, or, when said phenyl ring is substituted, there is a fluorine atom at the 3- or 4-position of the phenyl ring.

The compounds covered by the compound of formula (I) are:
methyl 2-({3-amino-2-phenylquinolin-4-yl}carbonyl)-1-phenylhydrazinecarboxylate,
methyl 2-({3-amino-2-[2-fluoro]phenylquinolin-4-yl}carbonyl)-1-phenylhydrazinecarboxylate,
methyl 2-({3-amino-2-[3-fluoro]phenylquinolin-4-yl}carbonyl)-1-phenylhydrazinecarboxylate,
methyl 2-({3-amino-2-[4-fluoro]phenylquinolin-4-yl}carbonyl)-1-phenylhydrazinecarboxylate,
methyl 2-({3-amino-2-[2,3-difluoro]phenylquinolin-4-yl}carbonyl)-1-phenylhydrazinecarboxylate,
methyl 2-({3-amino-2-[2,4-difluoro]phenylquinolin-4-yl}carbonyl)-1-phenylhydrazinecarboxylate,
methyl 2-({3-amino-2-[2,5-difluoro]phenylquinolin-4-yl}carbonyl)-1-phenylhydrazinecarboxylate,
methyl 2-({3-amino-2-[2,6-difluoro]phenylquinolin-4-yl}carbonyl)-1-phenylhydrazinecarboxylate,
methyl 2-({3-amino-2-[3,4-difluoro]phenylquinolin-4-yl}carbonyl)-1-phenylhydrazinecarboxylate,
methyl 2-({3-amino-2-[3,5-difluoro]phenylquinolin-4-yl}carbonyl)-1-phenylhydrazinecarboxylate,
and their pharmaceutically acceptable salts.

These compounds and those defined by the immediately preceding definitions are useful in therapy, especially as NK-2 and/or NK-3 antagonists, particularly as NK-3 antagonists.

The compounds of the present invention show advantageous properties. In particular, they possess one or more of the following characteristics as compared to prior art compounds:
(i) they provide a higher in *vivo* brain concentration of pharmacologically active compound after appropriate dosing;
(ii) they provide a higher *in vivo* brain:blood concentration ratio of pharmacologically active compound after appropriate dosing;
(iii) they achieve a higher level of NK-3 receptor occupancy at a given dose; this may mean that a lower dose would be needed for effective treatment of e.g. a CNS disorder than a compound with lower receptor occupancy;
(iv) they result in a lower level of adverse peripheral effects at a given dose;
(v) they have improved selectivity for the NK-3 receptor;
(vi) they have greater ability to effectively reverse behavioural effects driven by a NK-3 agonist *in vivo;*
(vii) they have superior properties in appropriate models predictive of therapeutic efficacy in CNS disorders or CNS-mediated disorders;
(viii) they have an improved duration of action;
(ix) they have other properties which make them superior candidates for development as medicaments, including physical characteristics which would aid their formulation.

The terms "administration of" and or "administering a" compound should be understood to mean providing a compound of the invention to the individual in need of treatment.

The term "subject," (alternatively referred to herein as "patient") as used herein refers to an animal, preferably a mammal, most preferably a human, who has been the object of treatment, observation or experiment.

The compounds of the present invention may be administered in the form of pharmaceutically acceptable salts. The term "pharmaceutically acceptable salt" is intended to include all acceptable salts such as acetate, lactobionate, benzenesulfonate, laurate, benzoate, malate, bicarbonate, maleate, bisulfate, mandelate, bitartrate, mesylate, borate, methylbromide, bromide, methylnitrate, calcium edetate, methylsulfate, camsylate, mucate, carbonate, napsylate, chloride, nitrate, clavulanate, N-methylglucamine, citrate, ammonium salt, dihydrochloride, oleate, edetate, oxalate, edisylate, palmoate (embonate), estolate, palmitate, esylate, pantothenate, fumarate, phosphate/diphosphate, gluceptate, polygalacturonate, gluconate, salicylate, glutamate, stearate, glycollylarsanilate, sulfate, hexylresorcinate, subacetate, hydrabamine, succinate, hydrobromide, tannate, hydrochloride, tartrate, hydroxynaphthoate, teoclate, iodide, tosylate, isothionate, triethiodide, lactate, panoate, valerate, and the like which can be used as a dosage form for modifying the solubility or hydrolysis characteristics or can be used in sustained release or pro-drug formulations. Depending on the particular functionality of the compound of the present invention, pharmaceutically acceptable salts of the compounds of this invention include those formed from cations such as sodium, potassium, aluminum, calcium, lithium, magnesium, zinc, and from bases such as ammonia, ethylenediamine, N-methyl-glutamine, lysine, arginine, ornithine, choline, N,N'-dibenzylethylene-diamine, chloroprocaine, diethanolamine, procaine, N-benzylphenethyl-amine, diethylamine, piperazine, tris(hydroxymethyl)aminomethane, and tetramethylammonium hydroxide. These salts may be prepared by standard procedures, e.g. by reacting a free acid with a suitable organic or inorganic base. Where a basic group is present, such as amino, an acidic salt, i.e. hydrochloride, hydrobromide, acetate, pamoate, and the like, can be used as the dosage form.

The compounds of the present invention may be administered by oral, parenteral (e.g., intramuscular, intraperitoneal, intravenous, ICV, intracistemal injection or infusion, subcutaneous injection, or implant), by inhalation spray, nasal, vaginal, rectal, sublingual, or topical routes of administration and may be formulated, alone or together, in suitable dosage unit formulations containing conventional non-toxic pharmaceutically acceptable carriers, adjuvants and vehicles appropriate for each route of administration. In addition to the treatment of warm-blooded animals such as mice, rats, horses, cattle, sheep, dogs, cats, monkeys, etc., the compounds of the invention are effective for use in humans.

The pharmaceutical compositions for the administration of the compounds of this invention may conveniently be presented in dosage unit form and may be prepared by any of the methods well known in the art of pharmacy. All methods include the step of bringing the active ingredient into association with the carrier which constitutes one or more accessory ingredients. In general, the pharmaceutical compositions are prepared by uniformly and intimately bringing the active ingredient into association with a liquid carrier or a finely divided solid carrier or both, and then, if necessary, shaping the product into the desired formulation. In the pharmaceutical composition the active object compound is included in an amount sufficient to produce the desired effect upon the process or condition of diseases. As used herein, the term "composition" is intended to encompass a product comprising the specified ingredients in the specified amounts, as well as any product which results, directly or indirectly, from combination of the specified ingredients in the specified amounts.

The pharmaceutical compositions containing the active ingredient may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the techniques described in the U.S. Patents 4,256,108; 4,166,452; and 4,265,874 to form osmotic therapeutic tablets for control release.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin, or olive oil.

Aqueous suspensions contain the active materials in admixture with excipients suitable for the manufacture of aqueous suspensions. Such excipients are suspending agents, for example sodium carboxymethylcellulose, methylcellulose, hydroxypropylmethylcellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

The pharmaceutical compositions of the invention may also be in the form of oil-in-water emulsions. The oily phase may be a vegetable oil, for example olive oil or arachis oil, or a mineral oil, for example liquid paraffin or mixtures of these. Suitable emulsifying agents may be naturally- occurring gums, for example gum acacia or gum tragacanth, naturally-occurring phosphatides, for example soy bean lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative and flavoring and coloring agents.

The pharmaceutical compositions may be in the form of a sterile injectable aqueous or oleagenous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

The compounds of the present invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

For topical use, creams, ointments, jellies, solutions or suspensions, etc., containing the compounds of the present invention are employed. (For purposes of this application, topical application shall include mouthwashes and gargles.)

The pharmaceutical composition and method of the present invention may further comprise other therapeutically active compounds as noted herein which are usually applied in the treatment of the above mentioned pathological conditions.

In the treatment or prevention of conditions which require NK-3 receptor modulation an appropriate dosage level will generally be about 0.01 to 500 mg per kg patient body weight per day which can be administered in single or multiple doses. Preferably, the dosage level will be about 0.1 to about 250 mg/kg per day; more preferably about 0.5 to about 100 mg/kg per day. A suitable dosage level may be about 0.01 to 250 mg/kg per day, about 0.05 to 100 mg/kg per day, or about 0.1 to 50 mg/kg per day. Within this range the dosage may be 0.05 to 0.5, 0.5 to 5 or 5 to 50 mg/kg per day. For oral administration, the compositions are preferably provided in the form of tablets containing 1.0 to 1000 milligrams of the active ingredient, particularly 1.0, 5.0, 10.0, 15.0, 20.0, 25.0, 50.0, 75.0, 100.0, 150.0, 200.0, 250.0, 300.0, 400.0, 500.0, 600.0, 750.0, 800.0, 900.0, and 1000.0 milligrams of the active ingredient for the symptomatic adjustment of the dosage to the patient to be treated. The compounds may be administered on a regimen of 1 to 4 times per day, preferably once or twice per day.

It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

The present invention also provides pharmaceutical compositions comprising a compound of formula I or a pharmaceutically acceptable salt thereof and a pharmaceutically acceptable excipient.

Thus there is provided a compound of formula I or a pharmaceutically acceptable salt thereof for use in therapy.

Likewise there is provided the use of a compound of formula I or a pharmaceutically acceptable salt thereof for the manufacture of a medicament for treating a neurokinin-2 and/or neurokinin-3 mediated disease.

There is also disclosed a method of treatment of a subject suffering from a neurokinin-2 and/or neurokinin-3 mediated disease, which comprises administering to that patient a therapeutically effective amount of a compound of formula I or a pharmaceutically acceptable salt thereof.

Examples of diseases mediated by neurokinin-2 and/or neurokinin 3 include CNS disorders such as depression (which term includes bipolar (manic) depression (including type I and type II), unipolar depression, single or recurrent major depressive episodes with or without psychotic features, catatonic features, melancholic features, atypical features (e.g. lethargy, over-eating/obesity, hypersomnia) or postpartum onset, seasonal affective disorder and dysthymia, depression-related anxiety, psychotic depression, and depressive disorders resulting from a general medical condition including, but not limited to, myocardial infarction, diabetes, miscarriage or abortion); anxiety disorders (including generalised anxiety disorder (GAD), social anxiety disorder (SAD), agitation, tension, social or emotional withdrawal in psychotic patients, panic disorder, and obsessive compulsive disorder); phobias (including agoraphobia and social phobia); psychosis and psychotic disorders (including schizophrenia, schizo-affective disorder, schizophreniform-diseases, acute psychosis, alcohol psychosis, autism, delirium, mania (including acute mania), manic depressive psychosis, hallucination, endogenous psychosis, organic psychosyndrome, paranoid and delusional disorders, puerperal psychosis, and psychosis associated with neurodegenerative diseases such as Alzheimer's disease); post-traumatic stress disorder; attention deficit hyperactive disorder (ADHD); cognitive impairment (e.g.the treatment of impairment of cognitive functions including attention, orientation, memory (memory disorders, amnesia, amnesic disorders and age-associated memory impairment) and language function, and including cognitive impairment as a result of stroke, Alzheimer's disease, Aids-related dementia or other dementia states, as well as other acute or sub-acute conditions that may cause cognitive decline such as delirium or depression (pseudodementia states)); convulsive disorders such as epilepsy (which includes simple partial seizures, complex partial seizures, secondary generalised seizures, generalised seizures including absence seizures, myoclonic seizures, clonic seizures, tonic seizures, tonic clonic seizures and atonic seizures); psychosexual dysfunction (including inhibited sexual desire (low libido), inhibited sexual arousal or excitement, orgasm dysfunction, inhibited female orgasm and inhibited male orgasm, hypoactive sexual desire disorder (HSDD), female sexual desire disorder (FSDD), and sexual dysfunction side-effects induced by treatment with antidepressants of the SSRI-class); sleep disorders (including disturbances of circadian rhythm, dyssomnia, insomnia, sleep apnea and narcolepsy); disorders of eating behaviours (including anorexia nervosa and bulimia nervosa); neurodegenerative diseases (such as Alzheimer's disease, ALS, motor neuron disease and other motor disorders such as Parkinson's disease (including relief from locomotor deficits and/or motor disability, including slowly increasing disability in purposeful movement, tremors, bradykinesia, hyperkinesia (moderate and severe), akinesia, rigidity, disturbance of balance and coordination, and a disturbance of posture), dementia in Parkinson's disease, dementia in Huntington's disease, neuroleptic-induced Parkinsonism and tardive dyskinesias, neurodegeneration following stroke, cardiac arrest, pulmonary bypass, traumatic brain injury, spinal cord injury or the like, and demyelinating diseases such as multiple sclerosis and amyotrophiclateral sclerosis); withdrawal from abuse of drugs including smoking cessation or reduction in level or frequency of such activities (such as abuse of cocaine, ethanol, nicotine, benzodiazepines, alcohol, caffeine, phencyclidine and phencyclidine-like compounds, opiates such as cannabis, heroin, morphine, sedative, hypnotic, amphetamine or amphetamine-related drugs such as dextroamphetamine, methylamphetamine or a combination thereof); pain (which includes neuropathic pain (including diabetic neuropathy; sciatica; non-specific lower back pain; multiple sclerosis pain; pain associated with fibromyalgia or cancer; AIDS-related and HIV-related neuropathy; chemotherapy-induced neuropathy; neuralgia, such as post-herpetic neuralgia and trigeminal neuralgia; sympathetically maintained pain and pain resulting from physical trauma, amputation, cancer, toxins or chronic inflammatory conditions such as rheumatoid arthritis and osteoarthritis; reflex sympathetic dystrophy such as shoulder/hand syndrome), acute pain (e.g. musculoskeletal pain, post operative pain and surgical pain), inflammatory pain and chronic pain, pain associated with normally non-painful sensations such as "pins and needles" (paraesthesias and dysesthesias), increased sensitivity to touch (hyperesthesia), painful sensation following innocuous stimulation (dynamic, static or thermal allodynia), increased, sensitivity, to noxious stimuli (thermal, cold, mechanical hyperalgesia), continuing pain sensation after removal of the stimulation (hyperpathia) or an absence of or deficit in selective sensory pathways (hypoalgesia), pain associated with migraine, and non-cardiac chest pain); certain CNS-mediated disorders such as emesis, irritable bowel syndrome, and non-ulcer dyspepsia; COPD, asthma, cough, gastro-oesophageal reflex induced cough, and exacerbated asthma; urinary incontinence; hypertension; and conditions associated with platelet hyperaggregability such as tissue ulceration, nephrotic syndrome, diabetes, migraine, coronary artery disease, pre-eclampsia and stroke. Preferably, the compounds of the invention are useful for the treatment of depression; anxiety disorders; phobias; psychosis and psychotic disorders; post-traumatic stress disorder; attention deficit hyperactive disorder (ADHD); withdrawal from abuse of drugs including smoking cessation or reduction in level or frequency of such activities; and irritable bowel syndrome. More preferably, the compounds of the invention are useful for the treatment of depression; anxiety disorders; phobias; and psychosis and psychotic disorders (especially schizophrenia, schizo-affective disorder, and schizophreniform diseases. Most preferably, the compounds of the invention are useful for the treatment of schizophrenia.

The compounds for use in the present invention are generally active in the following tests. They normally have an IC₅₀ of less than 1µM and preferably less than 100nM.

Details of the NK-2 receptor and its heterologous expression can be found in Gerard et al., J. Biol. Chem., 265: 20455-20462, 1990 and Huang et al., Biochem., 33: 3007-3013, 1994. The latter paper also contains details of mutant scanning.

Details of the NK-3 receptor and its heterologous expression can be found in Huang et al., BBRC, 1992, 184: 966-972 and Sadowski et al., Neuropeptides, 1993, 24: 317-319.

A membrane preparation is prepared as follows. A 10-layer cell factory is seeded with CHO cells stably expressing NK-3 receptors. The CHO cells are prepared in a triple T 175 flask in 11 growth medium which contains Iscore's modified Dulbecco's medium containing 10ml/l 200mM L-Glutamine, 10ml/l penicillin-streptomycin, one vial of hypoxanthine-thymidine 500x/l, 1mg/ml geneticin and 10% fetal bovine serum (inactivated). The cells are grown for 3 days in an incubator. The medium is washed off and the factory is rinsed twice with 400ml PBS (Ca, Mg-free). 400ml enzyme free dissoc. solution (EFDS) is added and the factory is maintained for 10 min at room temperature. The cells are dislodged and the suspension poured into 500ml centrifuge bottles. The process is repeated with 200ml EFDS and the mixtures pooled giving 6 bottles in all, which are spun in a centrifuge for 10 min at 2200 rpm.

The supernatants are aspirated and the residual cell pellets are frozen at -80° for 30 min to improve cell lysis and then resuspended in 40ml Tris with inhibitors per cell factory. The cells are homogenized in 40ml aliquots with 8 strokes of a glass-teflon grinder at setting 40. The homogenate is transferred to 50ml centrifuge tubes and placed on a rocker for 15 min at r.t. The homogenate is rehomogenised and held on ice if necessary before being centrifuged again as above.

The supernatant is transferred to Sorvall tubes for an SS-34 roter and held on ice.

40ml cold Tris with inhibitors is used to resuspend and combine the pellets which are again spun as above. The supernatants are again transferred to Sorvall tubes which, with those above, are spun at 18000 rpm for 20 min.

The supernatants are discarded and the pellets resuspended in a Storage Buffer consisting of 2.50ml 1M Tris pH7.4, 50µl 1000x protease inhibitors (4mg/ml leupeptin (Sigmo), 40mg/ml Bacitracin (Sigma) and 10mM phosphoranidon (Peninsula) all dissolved in water) plus 0.5ml 0.5M MnCl₂ made up to 50ml with H₂O_{dd}. A 10ml syringe is used with 20-, 23- and 25-gauge needles sequentially.

A Bradford protein assay in conducted on 2-10µl aliquots with BSA as standard before 500-1000µl aliquots are snap-frozen in liquid nitrogen for storage at -80°C.

The membrane binding assay is carried out as follows. The amount of membranes needed to specifically bind < 10% of ¹²⁵I-NeurokinB is predetermined. The frozen stocks are then diluted to allow addition in 50µl.

The test compounds are dissolved in DMSO. An automated apparatus (Tecan) is programmed to add 5µl of compound or DMSO, approximately 100,000 cpm of isotope in 20µl buffer which is prepared from 50µMTris, pH7.5, 150µM NaCl, bovine serum albumin to 0.02%, and protease inhibitors as in the storage buffer, made up as 0.5M stock, and 175µl assay buffer (as the storage buffer but containing 5µM MnCl₂ and without NaCl) into deep well Marsh boxes (Marsh Biomedical Products) in a 96-well format. Excess unlabelled competing peptide is added by hand for non-specific binding as indicated below. The binding reaction is initiated by adding 50µl of cell membranes. The tubes are incubated with shaking for 1h at r.t. and filtered on a Tomtec 96 well cell harvester using Mach III filtermats (Tomtec) or using either a Packard 96-well harvester or Tomtec 9600 using Unifilter GF/C (Packard), presoaked in 0.25% polyethyleneimine and washed five times with 1X wash buffer (0.1M.Tris, pH7.4 and 1M NaCl, 1X = 100ml of 10X stock per litre of cold distilled water). If using Unifilter plates, 60µl Microscint 20 (Packard) is added to each well and the plate is then heat-sealed before counting in a Packard Topcount. Alternatively the filters from the filtermat are placed in 75x100mm plastic tubes and counted on a Cobra gamma counter.

For the assay, typically 10µg of membrane is used at 25,000 cpm which is filtered over a Unifilter GF/C presoaked in 0.5% BSA.

Assays for binding at the neurokinin-2 receptor can be carried out in an analogous manner.

The compounds of the present invention can be readily prepared according to the following reaction schemes and examples, or modifications thereof. Starting materials can be made from procedures known in the art or as illustrated. In these reactions, it is also possible to make use of variants which are themselves known to those of ordinary skill in this art, but are not mentioned in greater detail. Furthermore, other methods for preparing compounds of the invention will be readily apparent to the person of ordinary skill in the art in light of the following reaction schemes and examples.

Abbreviations used in the instant specification, particularly the Schemes and Examples, include the following:
DMSO = dimethylsulfoxide; EDAC = 1-(3-dimethylamino)propyl-3-ethylcarbodiimide; Et₂O = diethyl ether; EtOAc = ethyl acetate; h = hour(s); HOBT = 1-hydroxy benzotriazole hydrate; sat'd = saturated aqueous; rt = room temperature; THF = tetrahydrofuran.

Compounds of formula I can be made by reacting a compound of formula II with methyl chloroformate: 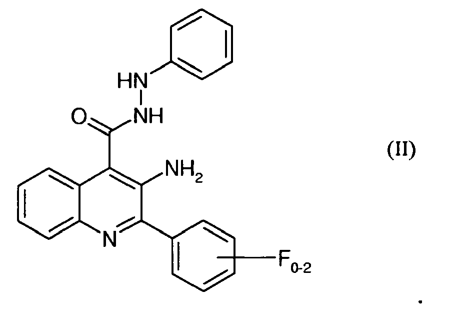 The reaction is generally carried out in a solvent such as toluene at raised temperature.

Compounds of formula II are generally known in the art or can be produced from known compounds by methods known in the art. For example, compounds of formula II can be made by reacting compounds of formula III with phenylhydrazine: 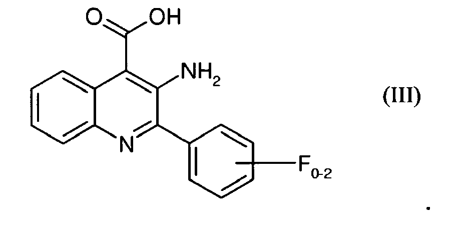 The reaction is generally carried out in a solvent such as THF in the presence of a base such as triethylamine and a condensing agent such as HOBT with EDAC.

Compounds of formula III are known in the art or can be made by known methods from known compounds.

The compounds of the present invention can be readily prepared according to the following examples, or modifications thereof. Starting materials can be made from procedures known in the art or as illustrated. In these reactions, it is also possible to make use of variants which are themselves known to those of ordinary skill in this art, but are not mentioned in greater detail. Furthermore, other methods for preparing compounds of the invention will be readily apparent to the person of ordinary skill in the art in light of the following examples. Unless otherwise indicated, the variables are as defined above.

The following Examples illustrate the present invention:

### Example 1: Methyl 2-({3-amino-2-phenylquinolin-4-yl}carbonyl)-1-phenylhydrazinecarboxylate

### Step 1: 3-Amino-N',2-diphenyl-4-quinolinecarbohydrazide

To a mixture of 3-amino-2-phenyl-quinoline-4-carboxylic acid (prepared according to the method of Giardina et al.; Journal of Heterocyclic Chemistry (1997), 34(2), 557-559; 0.95 g, 0.0036 mol), HOBT (0.84 g, 0.0056 mol) and triethylamine (0.78 ml, 0.0056 mol) in THF (100 ml) was added EDAC·HCl (1.04g, 0.0056 mol) followed by phenylhydrazine (0.39 ml, 0.004 mol) and the reaction mixture was stirred at rt for 16 h. All the solvent was removed *in vacuo* and the residue was dissolved in CH₂Cl₂ (100 ml) and washed with 2 M aqueous citric acid solution (2 x 60 ml), sat'd NaHCO₃ solution (2 x 60 ml) and saturated brine (1 x 60 ml). The organic phase was dried over anhydrous sodium sulfate, filtered and concentrated *in vacuo.* The residue was triturated with Et₂O and a yellow solid (0.9g) was collected by filtration. 1H NMR δ (ppm)(DMSO-d6, 500MHz): 10.50 (1H, d, *J* 2.8 Hz), 7.97 (1H, d, *J* 2.8 Hz), 7.90 (1H, d, *J* 7.7 Hz), 7.74 (3H, t, *J* 4.2 Hz), 7.60-7.46 (5H, m), 7.23 (2H, t, *J* 7.9 Hz), 6.89 (2H, d, *J* 7.6 Hz), 6.78 (1H, t, *J* 7.3 Hz), 5.10 (2H, s); MS: m/z shows MH+ = 355; C₂₂H₁₈N₅O requires M = 354.

### Step 2: Methyl 2-({3-amino-2-phenylquinolin-4-yl}carbonyl)-1-phenylhydrazinecarboxylate

To a solution of 3-amino-N',2-diphenyl-4-quinolinecarbohydrazide (from Step 1, 0.5 g, 0.00094 mol) in toluene (100 ml) was added methyl chloroformate (0.11 ml, 0.00141 mol) and the reaction mixture was heated at 60°C for 14h. A further amount of methyl chloroformate (0.45 ml) was added and heating continued for a further 24h and after this time a further amount of methyl chloroformate (0.45 ml) was added and heating continued for a further 6h. The solvent was removed *in vacuo* and the residue was dissolved in CH₂Cl₂ (100 ml) and washed with sat'd NaHCO₃ solution (2 x 50 ml), dried (Na₂SO₄), filtered and concentrated under vacuum. The residue was purified by chromatography on silica gel using 0-35% EtOAc in isohexane as eluent to give a solid which was recrystallised from Et₂O to give a yellow solid (0. 1 5g) which was collected by filtration. 1H NMR δ (ppm)(CDCl₃, 500MHz): 8.31 (1 H, s), 8.00 (1H, d, *J* 7.4 Hz), 7.88 (1H, s), 7.69 (2H, d, *J* 7.0 Hz), 7.56-7.42 (9H, m), 7.34 (1H, d, *J* 7.1 Hz), 5.17 (2H, s), 3.87 (3H, s); MS: m/z shows MH+ = 413; C₂₄H₂₀N₄O₃ requires M = 412.

### Example 2: Methyl 2-({3-amino-2-[4-fluoro]phenylquinolin-4-yl}carbonyl)-1-phenylhydrazinecarboxylate

### Step 1: 3-Amino-N'-phenyl-2-[4-fluoro]phenyl-4-quinolinecarbohydrazide

To a mixture of 3-amino-2-(4-fluoro-phenyl-quinoline)-4-carboxylic acid (prepared according to the method of published International patent application WO2004/050627; 0.7 g, 0.0026 mol), HOBT (0.5 g, 0.0039 mol) and triethylamine (0.52 ml, 0.0039 mol) in THF (100 ml) was added EDAC·HCl (0.713g, 0.0039 mol) followed by phenylhydrazine (0.295 ml, 0.003 mol) and the reaction mixture was stirred at rt for 16 h. All the solvent was removed *in vacuo* and the residue was dissolved in CH₂Cl₂ (100 ml) and washed with 2 molar aqueous citric acid solution (2 x 60 ml), sat'd NaHCO₃ solution (2 x 60 ml) and saturated brine (1 x 60 ml). The organic phase was dried over anhydrous sodium sulfate, filtered and concentrated *in vacuo.* The residue was triturated with Et₂O and a yellow solid (0.3g) was collected by filtration. 1H NMR δ (ppm)(CDCl₃, 500MHz): 8.06 (1H, dd, *J* 8.0 Hz), 7.96 (1H, d, *J* 8.4 Hz), 7.90 (1H, s), 7.72 (2H, dd, *J* 5.3, 8.6 Hz), 7.62-7.47 (3H, m), 7.33-7.29 (2H, m), 7.22 (2H, s), 6.99 (2H, t, *J* 8.7 Hz), 6.49 (1H, s), 4.91 (2H, s); MS: m/z shows MH+ = 373; C₂₂H₁₇FN₅O requires M = 372.

### Step 2: Methyl 2-({3-amino-2-[4-fluoro]phenylquinolin-4-yl}carbonyl)-1-phenylhydrazinecarboxylate

To a solution of 3-amino-N'-phenyl-2-[4-fluoro]phenyl-4-quinolinecarbohydrazide (from Step 1, 0.3 g, 0.0008 mol) in toluene (50 ml) was added methyl chloroformate (0.124 ml, 0.0016 mol) and the reaction mixture was heated at 60°C for 14h. A further amount of methyl chloroformate (0.062 ml) was added and heating continued for a further 24h and after this time a further amount of methyl chloroformate (0.062 ml) was added and heating continued for a further 6h. The solvent was removed *in vacuo* and the residue was dissolved in CH₂Cl₂ (100 ml) and washed with sat'd NaHCO₃ solution (2 x 50 ml), dried (Na₂SO₄ filtered and concentrated under vacuum. The residue was purified by chromatography on silica gel using 0-35% EtOAc in isohexane as eluent and then 0-15% EtOAc in CH₂Cl₂ as eluent to give a solid which was recrystallised from Et₂O to give a yellow solid (0.078g) which was collected by filtration. 1H NMR δ (ppm)(CDCl₃, 500MHz): 8.26 (1H, s), 7.99 (1H, d, *J* 9.2 Hz), 7.86 (1H, s), 7.71 (2H, dd, *J* 5.4, 8.6 Hz), 7.55 (2H, d, *J* 7.7 Hz), 7.49-7.43 (4H, m), 7.34 (1H, t, *J* 7.4 Hz), 7.22 (2H, m), 5.12 (2H, s), 3.87 (3H, s); MS: m/z shows MH+ = 431; C₂₄H₁₉FN₄O₃ requires M = 430.

### Example 3: Methyl 2-({3-amino-2-[3-fluoro]phenylquinolin-4-yl}carbonyl)-1-phenylhydrazinecarboxylate

### Step 1: 3-Amino-N'-phenyl-2-[3-fluoro]phenyl-4-quinolinecarbohydrazide

To a mixture of 3-amino-2-(3-fluoro-phenyl-quinoline)-4-carboxylic acid (prepared according to the method of published International patent application WO2004/050626; 1.4 g, 0.0052 mol), HOBT (1.0 g, 0.0078 mol) and triethylamine (1.04 ml, 0.0078 mol) in THF (200 ml) was added EDAC-HCl (1.426g, 0.0039 mol) followed by phenylhydrazine (0.59 ml, 0.006 mol) and the reaction mixture was stirred at rt for 16 h. All the solvent was removed *in vacuo* and the residue was dissolved in CH₂Cl₂ (200 ml) and washed with 2 molar aqueous citric acid solution (2 x 100 ml), sat'd NaHCO₃ solution (2 x 100 ml) and saturated brine (1 x 100 ml). The organic phase was dried over anhydrous sodium sulfate, filtered and concentrated *in vacuo.* The residue was triturated with Et₂O and a yellow solid (0.57g) was collected by filtration. 1H NMR δ (ppm)(CDCl₃): 8.05 (1H, d, *J* 8.0 Hz), 7.97 (1H, d, *J* 7.7 Hz), 7.88 (1H, s), 7.56-7.49 (4 H, m), 7.45 (1H, d, *J* 8.3 Hz), 7.32 (2H, t, *J* 7.8 Hz), 7.23-7.19 (1H, m), 6.99 (3H, m), 6.49 (1H, s), 4.94 (2H, s); MS: m/z shows MH+= 373; C₂₂H₁₇FN₅O requires M = 372.

### Step 2: Methyl 2-({3-amino-2-[3-fluoro]phenylquinolin-4-yl}carbonyl)-1-phenylhydrazinecarboxylate

To a solution of 3-amino-N'-phenyl-2-[3-fluoro]phenyl-4-quinolinecarbohydrazide (from Step 1, 0.55 g, 0.00147 mol) in toluene (50 ml) was added methyl chloroformate (0.32 ml, 0.0041 mol) and the reaction mixture was heated at 60°C for 14h. A further amount of methyl chloroformate (0.2 ml) was added and heating continued for a further 24h and after this time a further amount of methyl chloroformate (0.5 ml) was added and heating continued for a further 6h. The solvent was removed *in vacuo* and the residue was dissolved in CH₂Cl₂ (100 ml) and washed with sat'd NaHCO₃ solution (2 x 50 ml), dried (Na₂SO₄), filtered and concentrated under vacuum. The residue was purified by chromatography on silica gel using 0-15% EtOAc in CH₂Cl₂ as eluent to give a solid which was recrystallised from Et₂O to give a yellow solid (0.32g) which was collected by filtration. 1H NMR δ (ppm)(CDCl₃, 500MHz): 8.25 (1H, s), 8.00 (1H, d, *J* 9.2 Hz), 7.88 (1H, s), 7.56-7.43 (9H, m), 7.34 (1H, t, *J* 7.4 Hz), 7.20 (1H, m), 5.15 (2H, s), 3.88 (3H, s), MS: m/z shows MH+ = 431; C₂₄H₁₉FN₄O₃ requires M = 430.

## Claims

1. A compound of Formula (I): 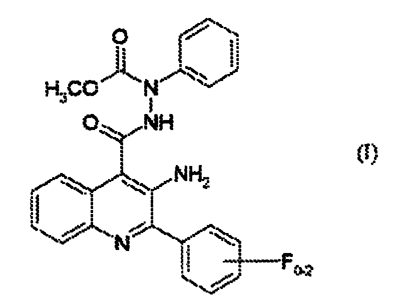 or a pharmaceutically acceptable salt thereof.

2. A compound as claimed in Claim 1 wherein the 2-position of the phenyl ring that is attached to the 2-position of the quinolinyl moiety is unsubstituted.

3. A compound as claimed in Claim 1 or Claim 2 wherein the phenyl ring that is attached to the 2-position of the quinolinyl moiety is unsubstituted.

4. A compound as claimed in Claim 1 selected from:
methyl 2-({3-amino-2-phenylquinolin-4-yl}carbonyl)-1-phenylhydrazinecarboxylate,
methyl 2-({3-amino-2-[2-fluoro]phenylquinolin-4-yl}carbonyl}-1-phenylhydrazinecarboxylate,
methyl 2-({3-amino-2-[3-fluoro]phenylquinolin-4-yl}carbonyl)-1-phenylhydrazinecarboxylate,
methyl 2-({3-amino-2-[4-fluoro]phenylquinolin-4-yl}carbonyl)-1-phenylhydrazinecarboxylate,
methyl 2-({3-amino-2-[2,3-difluoro] phenylquinolin-4-yl}carbonyl)-1-phenylhydrazinecarboxylate,
methyl 2-({3-amino-2-[2,4-difluoro]phenylquinolin-4-yl}carbonyl)-1-phenylhydrazinecarboxylate,
methyl 2-({3-amino-2-[2,5-difluoro]phenylquinolin-4-yl}carbonyl)-1-phenylhydrazinecarboxylate,
methyl 2-({3-amino-2-[2,6-difluoro]phenylquinolin-4-yl}carbonyl)-1-phenylhydrazinecarboxylate,
methyl 2-({3-amino-2-[3,4-difluoro]phenylquinolin-4-yl}carbonyl)-1-phenylhydrazinecarboxylate,
methyl 2-({3-amino-2-[3,5-difluoro]phenylquinolin-4-yl}carbonyl)-1-phenylhydrazinecarboxylate,
and pharmaceutically acceptable salts thereof.

5. A pharmaceutical composition comprising a compound of any one of Claims 1 to 4 or a pharmaceutically acceptable salt thereof and a pharmaceutically acceptable excipient.

6. A compound as claimed in any one of Claims to 4 for use in therapy.

7. The use of a compound of any one of Claims t to 4 or a pharmaceutically acceptable salt thereof for the manufacture of a medicament for treating a neurokinin-2 and/or neurokinin-3 mediated disease.

8. The use as claimed in Claim 7 wherein the neurokinin-2 and/or neurokinin-3 mediated disease is schizophrenia.

9. A process for preparing a compound of any one of Claims I to 4 or a pharmaceutically acceptable salt thereof by reacting a compound of formula II with methyl chloroformate:

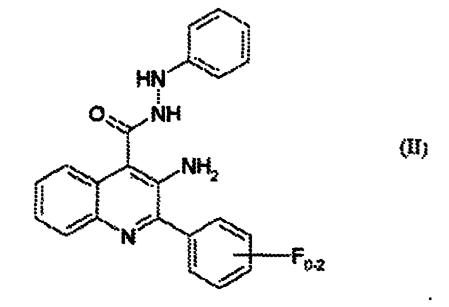

## Patentansprüche

1. Eine Verbindung der Formel (I): 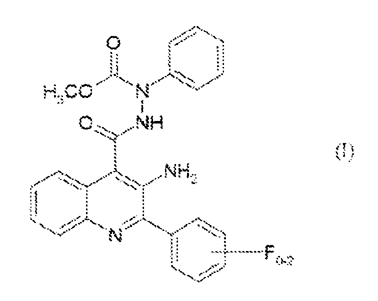 oder ein pharmazeutisch annehmbares Salz davon.

2. Eine wie in Anspruch 1 beanspruchte Verbindung, wobei die 2-Position des Phenylrings, der an die 2-Position des Chinolinylrestes gebunden ist, unsubstituiert ist.

3. Eine wie in Anspruch 1 oder Anspruch 2 beanspruchte Verbindung, wobei der Phenylring, der an die 2-Position des Chinolinylrestes gebunden ist, unsubstituiert ist.

4. Eine wie in Anspruch 1 beanspruchte Verbindung, ausgewählt aus:
Methyl-2-({3-amino-2-phenylchinolin-4-yl} carbonyl)-1-phenylhydrazincarboxylat,
Methyl-2-({3-amino-2-[2-fluor]phenylchinolin-4-yl} carbonyl)-1-phenylhydrazincarboxylat,
Methyl-2-({3-amino-2-[3-fluor]phenylchinolin-4-yl} carbonyl)-1-phenylhydrazincarboxylat,
Methyl-2-({3-amino-2-[4-fluor]phenylchinolin-4-yl} carbonyl)-1-phenylhydrazincarboxylat,
Methyl-2-({3-amino-2-[2,3-difluor]phenylchinolin-4-yl} carbonyl)-1-phenylhydrazincarboxylat,
Methyl-2-({3-amino-2-[2,4-difluor]phenylchinolin-4-yl} carbonyl)-1-phenylhydrazincarboxylat,
Methyl-2-({3-amino-2-[2,5-difluor]phenylchinolin-4-yl} carbonyl)-1-phenylhydrazincarboxylat,
Methyl-2-({3-amino-2-[2,6-difluor]phenylchinolin-4-yl} carbonyl)-1-phenylhydrazincarboxylat,
Methyl-2-({3-amino-2-[3,4-difluor]phenylchinolin-4-yl} carbonyl)-1-phenylhydrazincarboxylat,
Methyl-2-({3-amino-2-[3,5-difluor]phenylchinolin-4-yl} carbonyl)-1-phenylhydrazincarboxylat,
und pharmazeutisch annehmbare Salze davon.

5. Eine pharmazeutische Zusammensetzung, die eine Verbindung nach irgendeinem der Ansprüche 1 bis 4 oder ein pharmazeutisch annehmbares Salz davon und einen pharmazeutisch annehmbaren Hilfsstoff enthält.

6. Eine wie in irgendeinem der Ansprüche 1 bis 4 beanspruchte Verbindung zur Verwendung in der Therapie.

7. Die Verwendung einer Verbindung nach irgendeinem der Ansprüche 1 bis 4 oder eines pharmazeutisch annehmbaren Salzes davon zur Herstellung eines Medikaments zur Behandlung einer durch Neurokinin-2 und/oder Neurokinin-3 vermittelten Erkrankung.

8. Die wie in Anspruch 7 beanspruchte Verwendung, wobei die durch Neurokinin-2 und/oder Neurokinin-3 vermittelte Erkrankung Schizophrenie ist.

9. Ein Verfahren zur Herstellung einer Verbindung nach irgendeinem der Ansprüche 1 bis 4 oder eines pharmazeutisch annehmbaren Salzes davon durch Umsetzung einer Verbindung der Formel II mit Methylchlorformiat:

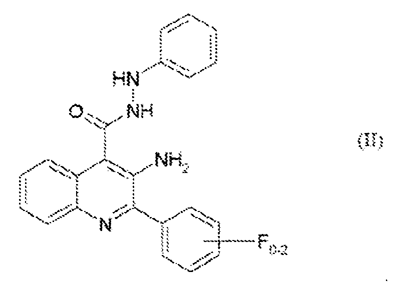

## Revendications

1. Composé selon la formule (I) : 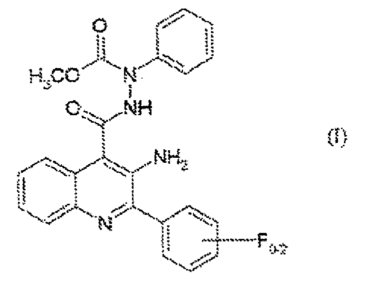 ou un sel pharmaceutiquement acceptable de celui-ci.

2. Composé tel que revendiqué dans la revendication 1, dans lequel la position 2 du cycle phényle qui est joint à la position 2 du fragment de quinolinyle est non substituée.

3. Composé tel que revendiqué dans la revendication 1 ou la revendication 2, dans lequel le cycle phényle qui est joint à la position 2 du fragment de quinolinyle est non substitué.

4. Composé tel que revendiqué dans la revendication 1 sélectionné à partir de :
méthyle 2-({3-amino-2-phénylquinoline-4-yl} carbonyle)-1-phénylhydrazinecarboxylate, méthyle 2-({3-amino-2-[2-fluoro]phénylquinoline-4-yl} carbonyle)-1-phénylhydrazinecarboxylate,
méthyle 2-({3-amino-2-[3-fluoro]phénylquinoline-4-yl} carbonyle)-1-phénylhydrazinecarboxylate,
méthyle 2-({3-amino-2-[4-fluoro]phénylquinoline-4-yl} carbonyle)-1-phénylhydrazinecarboxylate,
méthyle 2-({3-amino-2-[2,3-difluoro]phénylquinoline-4-yl}carbonyle)-1-phénylhydrazinecarboxylate,
méthyle 2-({3-amino-2-[2,4-difluoro]phénylquinoline-4-yl}carbonyle)-1-phénylhydrazinecarboxylate,
méthyle 2-({3-amino-2-[2,5-difluoro]phénylquinoline-4-yl}carbonyle)-1-phénylhydrazinecarboxylate,
méthyle 2-({3-amino-2-[2,6-difluoro]phénylquinoline-4-yl}carbonyle)-1-phénylhydrazinecarboxylate,
méthyle 2-({3-amino-2-[3,4-difluoro]phénylquinoline-4-yl}carbonyle)-1-phénylhydrazinecarboxylate,
méthyle 2-({3-amino-2-[3,5-difluoro]phénylquinoline-4-yl}carbonyle)-1-phénylhydrazinecarboxylate,
et de sels pharmaceutiquement acceptables de ceux-ci.

5. Composition pharmaceutique comprenant un composé selon l'une quelconque des revendications 1 à 4 ou un sel pharmaceutiquement acceptable de celui-ci et un excipient pharmaceutiquement acceptable.

6. Composé tel que revendiqué dans l'une quelconque des revendications 1 à 4 pour une utilisation en thérapie.

7. Utilisation d'un composé selon l'une quelconque des revendications 1 à 4 ou un sel pharmaceutiquement acceptable en vue de la fabrication d'un médicament afin de traiter une maladie induite par la neurokinine-2 et/ou la neurokinine-3.

8. Utilisation telle que revendiquée dans la revendication 7, dans laquelle la maladie induite par la neurokinine-2 et/ou la neurokinine-3 est la schizophrénie.

9. Procédé pour la préparation d'un composé selon l'une quelconque des revendications 1 à 4 ou un sel pharmaceutiquement acceptable de celui-ci en faisant réagir un composé de la formule II avec du chloroformate de méthyle :
